Europäisches Patentamt

European Patent Office

Office européen des brevets

(11) Veröffentlichungsnummer: **0 342 501**
**A2**

(12) **EUROPÄISCHE PATENTANMELDUNG**

(21) Anmeldenummer: 89108390.9

(22) Anmeldetag: 10.05.89

(51) Int. Cl.⁴ **C12Q 1/04 , G01N 1/30 ,**
**G01N 33/04**

(30) Priorität: 19.05.88 DE 3817089

(43) Veröffentlichungstag der Anmeldung:
23.11.89 Patentblatt 89/47

(84) Benannte Vertragsstaaten:
BE CH DE FR GB IT LI NL

(71) Anmelder: MERCK PATENT GESELLSCHAFT
MIT BESCHRÄNKTER HAFTUNG
Frankfurter Strasse 250 Postfach 4119
D-6100 Darmstadt(DE)

(72) Erfinder: Kroll, Werner, Dr.
Schwertstrasse 49
D-5630 Solingen(DE)
Erfinder: Rieke, Erwin, Dr.
Hermannstrasse 12
D-6104 Seeheim(DE)
Erfinder: Würzburg, Uwe, Dr.
Dessauer Strasse 12
D-6110 Dieburg(DE)

(54) Verfahren und Mittel zur Bestimmung von Bakterien und somatischen Zellen in Milch.

(57) Die Erfindung betrifft ein Verfahren und Mittel zur Bestimmung von Bakterien und somatischen Zellen in Milch. Das Verfahren ist dadurch gekennzeichnet, daß man die Milch mit einem Klärungsreagenz und mindestens einem Fluoreszenzfarbstoff behandelt und die fluoreszierenden Partikel durchflußcytometrisch oder mikroskopisch auswertet.

EP 0 342 501 A2

## Verfahren und Mittel zur Bestimmung von Bakterien und somatischen Zellen in Milch

Die vorliegende Erfindung betrifft ein Verfahren und Mittel zur Bestimmung von mikrobiellen und somatischen Zellen in Rohmilchproben, insbesondere ein Verfahren zur simultanen Messung beider Zelltypen ohne aufwendige Vorbehandlung.

Bei den bekannten Verfahren zur Bestimmung mikrobieller und somatischer Zellen werden diese jeweils getrennt in zwei Prozessen bestimmt, wobei die Bestimmung von Bakterien in Rohmilch vorzugsweise nach direkten Methoden erfolgt. Zu diesen Methoden gehören die auf mikrobiologischer Grundlage basierenden Koloniezählungen und mikroskopische Verfahren. Beim Verfahren der Mikrokoloniezählung werden Rohmilchproben entweder in oder auf ein Agar-Gel gegeben, inkubiert und die Anzahl sich bildender Makrokolonien gezählt. Die Anzahl der in der Rohmilch enthaltenen Bakterien ist proportional der Anzahl vorgefundener Makrokolonien. Bei der direkten mikroskopischen manuellen Zählung wird eine bestimmte Menge Rohmilch auf einem Objektträger ausgestrichen, eintrocknen gelassen und anschließend mit einer Färbelösung angefärbt. Die gefärbten Bakterien werden dann manuell gezählt. Nach einem abgewandelten Verfahren wird die Milch mit einer proteolytischen Enzymlösung vorbehandelt, über ein Filter abgesaugt und die auf dem Filter verbleibenden Bakterien angefärbt; die angefärbten Bakterien werden manuell ausgezählt.

Eine Automatisierung der mikroskopischen Zählung erfolgt nach der sogenannten BACTOSCAN-Methode, bei der die Rohmilchprobe zunächst mit einer Lysierlösung versetzt wird, damit Proteinpartikel und somatische Zellen zerfallen. Durch Gradientenzentrifugation zwischen zwei Medien (Sucrose und Dextran) werden die Bakterien von den restlichen Bestandteilen der Milch abgetrennt. Die Bakterienlösung wird aus der Zentrifuge abgezogen und mit einer proteolytischen Lösung von restlichen Proteinpartikeln befreit. Die verbleibenden Bakterien werden anschließend mit Acridinorange gefärbt. Ein Aliquot der Baktierensuspension wird als sehr dünner Film auf die Kante einer rotierenden Scheibe aufgebracht. Die Bestrahlung des Films mit Xenon-Licht läßt die Bakterien rotorange fluoreszieren. Das so erzeugte Fluoreszenzlicht wird über einen Fotodetektor empfangen und in entsprechende Impulse umgewandelt, die gezählt werden (EP-OS 8826).

Für die Zählung somatischer Zellen in Rohmilch finden vier Verfahren eine breite Anwendung. Dabei handelt es sich um die klassische mikroskopische Zählung, die Zählung nach dem Coulter-Verfahren, die Zählung nach der FossomaticMethode und die Verwendung eines Autoanalyzers zur Zellzählung. Zur mikroskopischen Zählung somatischer Zellen wird eine definierte Menge Milch auf einen Objektträger gegeben, der Film wird getrocknet, gefärbt und anschließend ausgezählt. Von einer Probe müssen mindestens 2 Filme aus gezählt werden. Nach dem Coulter-Verfahren wird die Probe zunächst mit Formalin fixiert, erhitzt und mit verschiedenen Lösungen zum Fettabbau behandelt. Nach entsprechender Verdünnung wird die Probe dem Meßgerät zugeführt und konduktometrisch ausgewertet.

Das Fossomatic-Verfahren benötigt eine mit Puffer vorverdünnte und mit Fluoreszenzfarbstoffen gefärbte Rohmilchprobe, die in feinem Strahl auf eine rotierende Scheibe gespritzt wird, die als Objektebene für ein automatisiertes Fluoreszenzmikroskop dient. Angefärbte Zellen erzeugen Flureszenzlicht, das elektronisch erfaßt wird. Die dadurch hervorgerufenen Meßimpulse werden gezählt und sind ein Maß für die Anzahl somatischer Zellen in der Probe. Das im Autoanalyzer verwendete Prinzip basiert auf der indirekten Methode der Streulichtmessung. Das in der Milch enthaltene Fett wird chemisch aufgelöst und das Streulicht gemessen, das durch die somatischen Zellen hervorgerufen wird.

Bei den bekannten Verfahren zur Bestimmung bakterieller und somatischer Zellen ist die Bestimmung grundsätzlich nur in getrennten Analysen möglich; sie sind kompliziert und zeitaufwendig. So liegen die Ergebnisse beim Koloniezählverfahren erst nach 48 Stunden vor. Die Bestimmung nach dem Bactoscan-Verfahren hat den Nachteil, daß die Milchprobe einer aufwendigen und wenig reproduzierbaren Vorbehandlung unterzogen werden muß und die Bakterienzahlen im Bereich unterhalb 50000/ml nicht mehr exakt bestimmt werden können. Die somatischen Zellen müssen in einer getrennten Messung nach dem Coulter- oder dem Fossomatic-Verfahren bestimmt werden.

Der Erfindung liegt die Aufgabe zugrunde, ein Verfahren und Mittel zur gleichzeitigen Bestimmung von Bakterien und somatischen Zellen in Milch zur Verfügung zu stellen, mit dem die oben genannten Nachteile der bekannten Verfahren vermieden werden können.

Gegenstand der Erfindung ist ein Verfahren zur Bestimmung von Bakterien und somatischen Zellen in Milch, das dadurch gekennzeichnet ist, daß man die Milch mit einem Klärungsreagenz und mindestens einem Flureszenzfarbstoff behandelt und die fluoreszierenden Partikel durchflußcytometrisch oder mikroskopisch auswertet.

Ein weiterer Gegenstand der Erfindung ist ein Mittel zur Durchführung dieses Verfahrens, das ein

Klärungsreagenz und ein Farbreagenz enthält.

Überraschenderweise hat sich gezeigt, daß durch eine einfache Vorbehandlung der Milch mit einem speziellen Klärungsreagenz und anschließender Sichtbarmachung der Bakterien und somatischen Zellen durch Färbung mit Fluoreszenzfarbstoffen, beide Zelltypen gleichzeitig bestimmt werden können. Das Klärungsreagenz bewirkt das selektive Auflösen aller partikulären Bestandteile der Milch, die nicht Zellen sind, wie Fett- und Albuminmicellen. Mit dem Farbreagenz werden dann die zellulären Partikel (Bakterien und somatische Zellen) angefärbt und über Meßsignale oder mikroskopisch ausgewertet. Auf diese Weise werden Störsignale vermieden und nur die für die Bestimmung interessierenden Partikel angefärbt. Das Verfahren erlaubt es, auch relativ kleine Mengen mikrobieller und somatischer Zellen in der Probe exakt auszuzählen.

Das erfindungsgemäße Klärungsreagenz baut die nicht zellulären Partikel der Rohmilchprobe ab, ohne die vorhandenen mikrobiellen und somatischen Zellen in ihrer Färbeeigenschaft gegenüber dem Farbstoff zu beeinträchtigen. Das Klärungsreagenz enthält im wesentlichen ein nichtionisches Detergenz, einen Alkohol mit bis zu 8 C-Atomen und einen Puffer vom pH-Wert > 10.

Als nichtionische Detergenzien eignen sich z.B. solche auf der Basis alkoxylierter Fettalkohole, Alkylphenole, Ethylendiamin, Fettsäureamide oder Fettamine, vorzugsweise Nonylphenolpolyethylenglycolether, Decylpolyethylenglycolether, Tridecylpolyethylenglycolether, Pentadecylpolyethylenglycolether, Talgfett mit 80 Mol Ethylenglycol.

Die nichtionischen Detergenzien können auch als Mischung eingesetzt werden. Die Konzentration sollte im Bereich von 100 bis 700 mg/ml des Klärungsreagenzes liegen, vorzugsweise im Bereich von 250 bis 450 mg/ml.

Als Alkohole eignen sich alle mit bis zu 8 C-Atomen, vorzugsweise solche mit 3 bis 7 C-Atomen wie Propanole, Butanole, Pentanole, Hexanole und Heptanole, vorzugsweise Pentanole, insbesondere 2-Pentanol. Die Konzentration des eingesetzten Alkohols bzw. Alkoholgemischs sollte im Bereich von 50 bis 500 mg/ml des Klärungsreagenzes liegen, vorzugsweise im Bereich von 200 bis 300 mg/ml.

Zur Aufrechterhlatung eines pH-Wertes > 10 erhält das erfindungsgemäße Klärungsreagenz geeignete Puffersubstanzen wie Tris-Puffer, Phosphatpuffer, Boratpuffer, CHES-Puffer, CAPS-Puffer bzw. eine Lauge, wie Natronlauge, Kalilauge, primäre, sekundäre oder tertiäre Amine.

Die Konzentration des Puffers sollte im Bereich von 200 bis 700 mg/ml des Klärungsreagenzes liegen, vorzugsweise im Bereich von 350 bis 500 mg/ml.

Das Farbreagenz nach der Erfindung enthält mindestens einen Fluoreszenzfarbstoff. Geeignete Farbstoffe sind z.B. Ethidiumbromid, Acridinorange, Propidiumjodid, Dimidiumbromid, Carbocyanine, Thioflavine usw. Vorzugsweise enthält das Farbreagenz zwei Farbstoffe. Die Konzentration der Farbstoffe im Farbreagenz beträgt zweckmäßigerweise 1 bis 500 mg/l, vorzugsweise 10 bis 150 mg/l.

Zur Durchführung des Verfahrens werden das Klärungsreagenz, das Farbreagenz und die zu untersuchende Rohmilch auf eine Temperatur von 40-60 °C gebracht. 500 bis 1000 ml vortemperiertes Klärungsreagenz werden in einem Reaktionsgefäß vorgelegt und unter Rühren werden 200 bis 600 µl Rohmilch hinzupipettiert. Nach kurzer Zeit (einige Sekunden) ist die Lösung vollständig klar; Fett- und Proteinmicellen sind aufgelöst und die in der Rohmilch vorhandenen Zellen durch das Klärungsreagenz in ihrer Membran geschädigt. Für niedermolekulare Reagenzien ist die Membran der Zellen nun vollkommen durchlässig. Im Anschluß an diese Vorbehandlung werden 50-200 µl der Farbstofflösung hinzugegeben und die vorgeschädigten Zellen angefärbt. Die Anfärbung erfolgt durch intensives Vermischen von Probe und Reagenz. Alternativ zu diesem Vorgehen kann das Farbreagenz auch direkt dem Klärungsreagenz zugesetzt werden und Vorbehandlung und Färbung der in der Milch enthaltenen Partikel in einem Einschrittverfahren vorgenommen werden. Anstatt die einzelnen Reagenzien und die Probe in einem geeigneten Reaktiongefäß zu vermischen, können die Reagenzien auch über entpsrechende Schlauchsysteme zusammengebracht werden.

Im Anschluß hieran ist die Probe meßbereit. In einem Durchflußzytometer kann die Fluoreszenzintensität der zellulären Partikel quantifiziert und ausgewertet werden. Man kann dabei so vorgehen, daß man entweder einparametrig oder zweiparametrig, d.h. in zwei verschiedenen Emissionsbereichen (z.B. 500-560 nm und 570-690 nm) die Fluoreszenzintensität der zellulären Partikel von Einzelzellen erfaßt. Die erhaltenen Histogramme werden in üblicher Weise ausgewertet.

Alternativ dazu ist auch eine Auszählung von Bakterien und somatischen Zellen unter dem Mikroskop möglich. Bakterien und somatische Zellen erscheinen im Fluoreszenzlicht intensiv angefärbt. Durch ihre unterschiedliche Größe sind sie unter dem Mikroskop einwandfrei zu unterscheiden.

Beispiel 1

Im Wasserbad werden ein Klärungsreagenz aus

| 4 Teilen | Tridecylpolyethylenglycolether (Lutensol® AO), |
|---|---|
| 1 Teil | 2-Pentanol, |
| 2 Teilen | Natronlauge 0,1 M |

und Rohmilch auf 40 °C temperiert. In einem Reagenzröhrchen werden zu 1 ml des Klärungsreagenzes unter kräftigem Rühren 400 µl Milch hinzugegeben. Nach dem Aufklären der Lösung (ca. 5 Sekunden) werden 100 µl einer Farbstofflösung aus

| 100 mg/l | Propidiumjodid und |
|---|---|
| 20 mg/l | Dihexyloxacarbocyaninjodid in |
| 50 mM | Tris-Puffer (pH 7,0) |

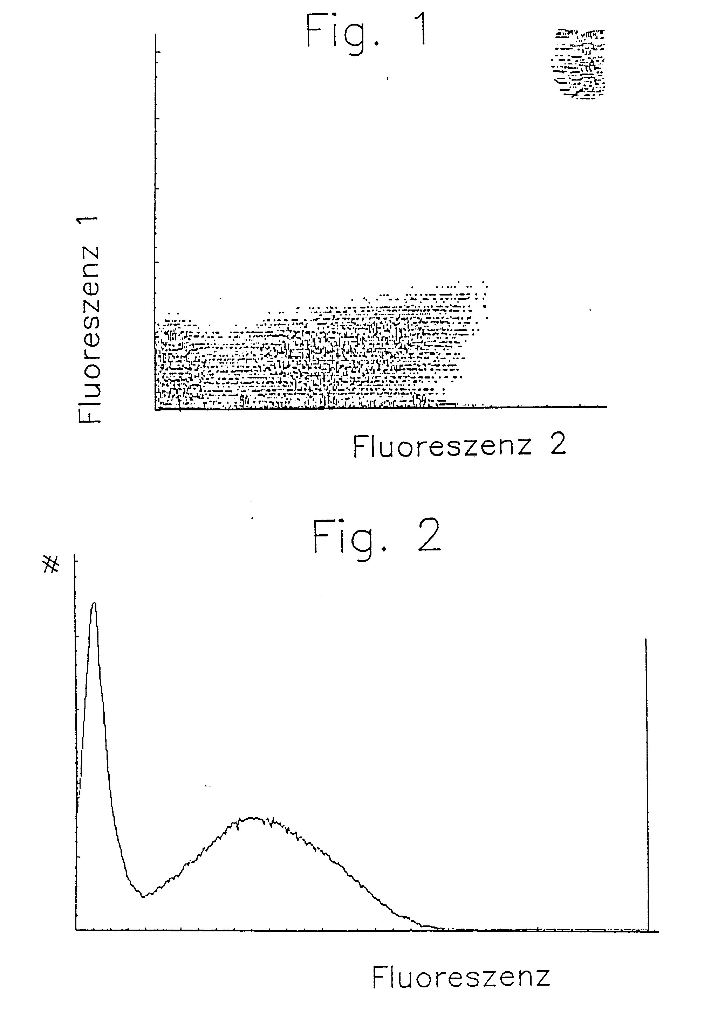

zu der Lösung hinzugegeben. Die Fluoreszenzintensität der erhaltenen Lösung wird in einem Durchflußzytometer bei zwei verschiedenen Emissionbereichen (500-560 nm und 570-690 nm) erfaßt. Man erhält ein zweidimensionales Histogramm, in dem zwei deutliche Cluster zu erkennen sind (Fig. 1).

Das Cluster im Bereich niedriger Fluoreszenz 1 und Fluoreszenz 2 repräsentiert die Bakterien, das mit jeweils hoher Intensität Fluoreszenz 1 und Fluoreszenz 2 die somatischen Zellen. Eine Summation der Partikel innerhalb der jeweiligen Cluster ergibt die Anzahl der Bakterien und somatischen Zellen in der Milchprobe wieder.

Beispiel 2

Analog Beispiel 1 werden ein Klärungsreagenz aus

| 4 Teilen | Nonylphenolpolyethylenglycolether (Lutensol® AP 14), |
|---|---|
| 3 Teilen | 2-Pentanol, |
| 5 Teilen | Phosphatpuffer, 0,1 M (pH 10,5) |

Rohmilch und eine Farbstofflösung aus

| 10 mg/l | Ethidiumbromid in |
|---|---|
| 20 mM | Phosphatpuffer (pH 7,0) |

zusammengegeben. Die fluorochromierte Probe wird in einem Durchflußzytometer einparametrig gemessen. Man erhält ein Histogramm mit zwei Maxima (Fig. 2). Das erste Maximum repräsentiert die bakteriellen Zellen, das zweite Maximum die somatischen Zellen. Eine Integration der Kurven ergibt den Gehalt der beiden Zellarten.

Beispiel 3

In einem Wasserbad werden ein Klärungsreagenz aus

| 3 Teilen | Talgfett-Polyethylenglycolether (Lutensol® A 80), |
|---|---|
| 2 Teilen | Isobutanol, |
| 5 Teilen | Kalilauge 0,1 M |

und Rohmilch auf 40 °C temperiert. In einem Reagenzröhrchen werden zu 1 ml des Klärungsreagenzes unter starkem Rühren 400 µl Milch hinzugegeben. Nach dem Aufklären der Lösung werden 100 µl einer Farbstofflösung aus 50 mg/l Dimidiumbromid in 30 mM Puffer zu der Lösung hinzugegeben.

Auf diese Weise wurden 40 verschiedene Rohmilchproben mit unterschiedlichem Gehalt an bakteriellen und somatischen Zellen durchflußcytometrisch gemessen. Von den gleichen Proben wurde der Gehalt an Bakterien auch nach dem Plattengußverfahren und der Gehalt an somatischen Zellen nach dem Fossomatic-Verfahren ermittelt. Ein Vergleich der Methoden zeigt eine sehr gute Übereinstimmung der Ergebnisse.

Beispiel 4

In einem Wasserbad werden ein Klärungsreagenz aus

| | |
|---|---|
| 3 Teilen | Decylpolyethylenglycolether (Lutensol® ON 110), |
| 2 Teilen | Isobutanol, |
| 5 Teilen | Diethylamin |

und Rohmilch auf 30 °C temperiert. In einem Reagenzröhrchen werden zu 1 ml des Klärungsreagenzes unter starkem Rühren 200 $\mu$l Milch hinzugegeben. Nach dem Aufklären der Lösung werden 100 $\mu$l einer Farbstofflösung aus 50 mg/l Thioflavin TCN in 50 mM Tris-Puffer zu der Lösung hinzugegeben.

Sofort nach Durchmischen der Flüssigkeiten wird eine Neubauer-Zählkammer an jeder Seite mit je einem Tropfen des Gemisches beschickt. Man läßt die Zellen sedimentieren und zählt dann am Mikroskop die fluoreszierenden Zellen aus.

Beispiel 5

Ein Klärungsreagenz aus

| | |
|---|---|
| 8 Teilen | Decylpolyethylenglycolether (Lutensol® ON 110), |
| 5 Teilen | 3-Pentanol, |
| 8 Teilen | Triethylamin |

wird auf 50 °C temperiert. Zu 1 ml dieses Reagenzes werden 300 $\mu$l Rohmilch (Raumtemperatur) gegeben und kräftig geschüttelt. Nach dem Aufklären der Lösung werden 100 $\mu$l einer Farbstofflösung aus

| | |
|---|---|
| 25 mg/l | Acridinorange in |
| 50 mM | Trispuffer (pH 7,5) |

zu der Lösung hinzugegeben.

Die Fluoreszenzintensität der erhaltenen Lösung wird in einem Durchflußcytometer im Emissionsbereich > 520 nm erfaßt. Man erhält ein eindimensionales Histogramm, in dem die Bakterien bei niedriger und die somatischen Zellen bei hoher Fluoreszenzintensität erscheinen. Die Summation der relevanten Bereiche in dem Pulshöhenhistogramm ergibt die Anzahl der Bakterien und somatischen Zellen je ml vermessener Rohmilchprobe.

**Ansprüche**

1. Verfahren zur Bestimmung von Bakterien und somatischen Zellen in Milch, dadurch gekennzeichnet, daß man die Milch mit einem Klärungsreagenz und mindestens einem Fluoreszenzfarbstoff behandelt und die fluoreszierenden Partikel durchflußcytometrisch oder mikroskopisch auswertet.

2. Mittel zur Durchführung des Verfahrens nach Anspruch 1, enthaltend ein Klärungsreagenz und ein Farbreagenz.

3. Mittel nach Anspruch 2, dadurch gekennzeichnet, daß das Klärungsreagenz im wesentlichen ein nichtionisches Detergenz, einen Alkohol mit bis zu 8 C-Atomen und einen Puffer vom pH-Wert > 10 enthält.

4. Mittel nach Anspruch 2, dadurch gekennzeichnet, daß das Farbreagenz Ethidiumbromid, Acridinorange, Propidiumjodid, Dimidiumbromid, ein Carbocyanin und/oder ein Thioflavin enthält.

# Fig. 1

Fluoreszenz 1

Fluoreszenz 2

# Fig. 2

\#

Fluoreszenz